# FASCICULE DE BREVET EUROPEEN

(11) **EP 0 608 172 B1**
(45) Date de publication et mention de la délivrance du brevet: **04.04.2001**
(21) Numéro de dépôt: 94400107.2
(22) Date de dépôt: 18.01.1994
(51) Int. Cl.: C12P 23/00, C12N 1/16, C12P 7/26, A61K 7/00, A61K 31/015, A23L 1/303, A23K 1/16, C12P 1/02

(54) **Mutants de Phaffia Rhodozyma, procédé de production de beta-carotène et utilisation de biomasse riche en beta-carotène**
Phaffia rhodozyma Mutanten, Verfahren zur Herstellung von Beta-Carotin und Verwendung von einer Beta-Carotin-reichen Biomasse
Phaffia rhodozyma mutants, process for the production of beta-caroten and use of a beta-caroten rich biomass

(30) Priorité: 19.01.1993 FR 9300463
(43) Date de publication de la demande: 27.07.1994
(73) Titulaire: PERNOD-RICARD, 75008 Paris (FR)
(72) Inventeur: Girard, Patrick, F-91940 Les Ulis (FR); Javelot, Catherine Eliane Jeannie, F-44300 Nantes (FR); Vladescu, Barbu Dinu Vladimir, F-75016 Paris (FR)
(74) Mandataire: Warcoin, Jacques

(56) Documents cités:
- APPLIED AND ENVIRONMENTAL MICROBIOLOGY vol. 56, no. 9 , Septembre 1990 , AMERICAN SOCIETY FOR MICROBIOLOGY pages 2944 - 2945 LEWIS, M.J. ET AL. 'Selection of astaxanthin-overproducing mutants of Phaffia rhodozyma with beta-ionone'
- THE MERCK INDEX, 11th edition, 1989 page 282, entry number 1860
- DATABASE WPI Section Ch, Week 8530, Derwent Publications Ltd., London, GB; Class B04, AN 85-181407 & JP-A-60 110 285 (NIPPON CARBIDE KOGY KK) 15 Juin 1985
- CHEMICAL ABSTRACTS, vol. 72, no. 13, 30 Mars 1970, Columbus, Ohio, US; abstract no. 65371c, LAROE, E. & SHIPLEY, P. 'Whisky composition: formation of alpha- and beta-ionone by the thermal decomposition of beta-carotene' page 250 ; & J.AGR.FOOD CHEM., 1970, 18(1); 174-5
- APPLIED AND ENVIRONMENTAL MICROBIOLOGY vol. 55, no. 1 , Janvier 1989 , AMERICAN SOCIETY FOR MICROBIOLOGY pages 116 - 124 AN, G.-H. ET AL. 'Isolation of Phaffia rhodozyma mutants with increased astaxanthin content'
- "Biotechnology of vitamins, pigments and growth factors", Vandamme, E.J. (ed.), Elsevier Applied Science, London, 1989.

## Description

La présente invention concerne des mutants de Phaffia rhodozyma bloqués dans la voie de la carotenogenèse, des procédés d'obtention de β-carotène, l'utilisation de biomasse de ces mutants et l'utilisation de β-carotène.

Principalement connu comme la plus importante des provitamines A, (+ Le Merck Index, 11ème édition, 1989, p. 282, n° d'entrée 1860) le β-carotène fait actuellement l'objet d'études épidémiologiques et cliniques approfondies qui semblent lui attribuer des rôles physiologiques autres que ceux liés à la vitamine A.

En effet, la conversion du β-carotène en vitamine A dans la paroi intestinale étant régulée par le taux même de vitamine A dans le sang, une partie du β-carotène alimentaire reste à l'état de provitamine et exerce une fonction antioxydante vraisemblablement responsable de la protection contre certaines formes de cancer, athérosclérose, arthrite rhumatoïde, cataracte sénile, parkinsonisme. Une dose journalière de 5,2 à 6,0 mg de β-carotène dans les aliments a été préconisée comme facteur important de prévention de ces maladies.

Comme le β-carotène est également un pigment naturel susceptible de remplacer certains colorants de synthèse et qu'il jouit d'une perception très favorable de la part des Consommateurs, des efforts considérables se portent actuellement sur l'élaboration de nouveaux produits alimentaires contenant du β-carotène, produits faisant partie de la catégorie des aliments de nouvelle génération, dits aliments "fonctionnels". Par ailleurs, il semblerait que le β-carotène soit un agent de protection solaire très efficace, d'où certaines applications dans l'industrie des produits cosmétiques.

Bien que les caroténoïdes en général et le β-carotène en particulier soient largement répandus dans le monde vivant, seulement les plantes et certains microorganismes possèdent les équipements enzymatiques nécessaires à leur biosynthèse à partir d'acétyl-CoA, via l'acide mévalonique.

Parmi les microorganismes, certains champignons et algues synthétisent des carotènes (dont le β-carotène), alors que les bactéries produisent essentiellement des xanthophyles. Les procédés de production de β-carotène en fermenteur proposés jusqu'à présent utilisent l'algue *Dunaliella (Ben Amotz & Avron*, 1980) et les phycomycètes *Phycomyces* *blakesleeanus (Murillo-Araujo et al*, 82) et *Blakeslea trispora (Ninet et Renaut*, 1979).

Quant aux levures, à une exception près, ces microorganismes ne font pas l'objet de procédés industriels de production de caroténoïdes. Le torulène, synthétisé par des levures rouges appartenant aux genres *Rhodotorula* et *Rhodosporidium* (Database WPI, Semaine 8530, AN85-181407), ne présente qu'un intérêt mineur, alors que les autres caroténoïdes identifiés chez les levures (dont le β-carotène) sont présents en d'aussi faibles quantités qu'une exploitation industrielle semble peu envisageable.

Le seul exemple de caroténoïde produit industriellement par emploi de levures est offert par l'astaxanthine, pigment caractéristique de la levure Phaffia rhodozyma. Après optimisation de la production, l'astaxanthine ou la biomasse levurienne sont utilisés comme supplément dans l'alimentation du saumon, de la truite saumonnée, des crustacés, des poules pondeuses, etc... (Brevet WO 91/02060).

Les caroténoïdes autres que le β-carotène ne présentent ni le potentiel vitaminique du β-carotène, ni son effet protecteur antioxydant. Cependant, le rendement important du fonctionnement de la voie de biosynthèse de l'astaxanthine chez Phaffia rhodozyma, ainsi que l'alimentarité virtuelle de cettte espèce nous ont suggéré de mettre à profit les propriétés de cette levure dans un procédé de production de β-carotène.

Une production de β-carotène par levures serait donc intéressante car celles-ci sont, d'une part, plus faciles à cultiver en masse que d'autres microorganismes tels que les champignons et les plantes et, d'autre part, la biomasse de mutants de levures producteurs de β-carotène pourrait être utilisée telle quelle dans les produits notamment alimentaires, diététiques cosmétiques, parapharmaceutiques ou pharmaceutiques.

L'effet de la β-ionone sur la production de β-carotène par une souche sauvage et une souche mutée de Phaffia rhodozyma a été décrit dans Lewis et al., Applied and Environmental Microbiology, Vol. 56, n° 9, septembre 1990, 2944-2945. On sait par ailleurs que la décomposition thermique de β-carotène donne lieu à la formation de α et β-ionone (Chemical Abstract. Vol. 72, n° 13, 30 mars 1970, n° 65371c, Laroe et al.)

La présente invention fournit des mutants de Phaffia rhodozyma bloqués dans la voie de la caroténogénèse et qui accumulent du β-carotène.

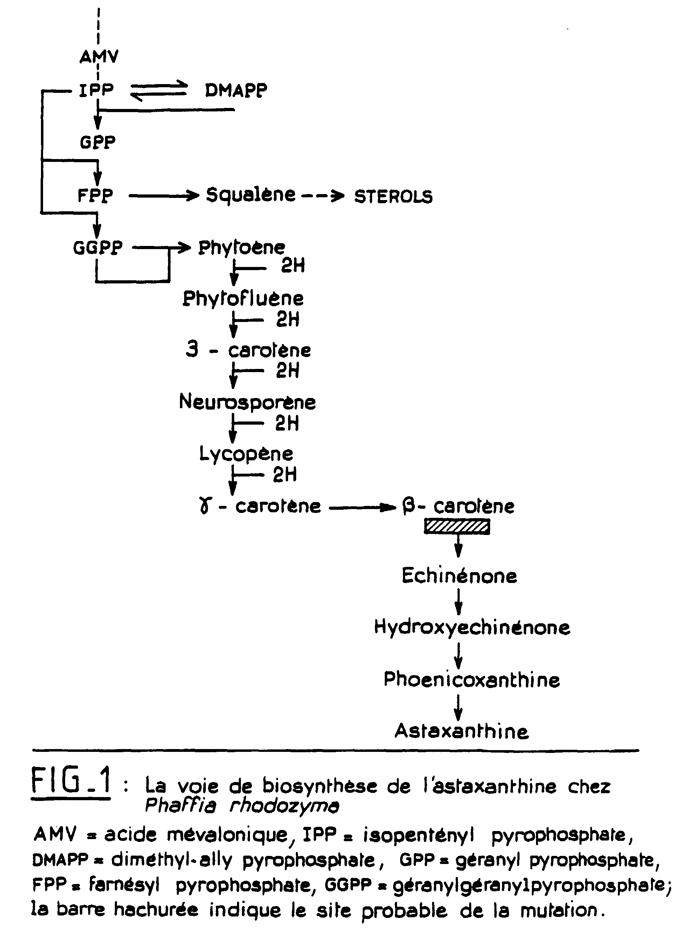

La voie de biosynthèse de l'astaxanthine chez Phaffia rhodozyma a été en partie élucidée et est présentée à la Figure 1.

Les mutants selon l'invention sont bloqués vraisemblablement dans l'étape de conversion du β-carotène en echinénone, notamment par traitement aux UV. Ces mutants sont incapables de produire de l'astaxanthine.

La présente invention a en effet pour objet Procédé d'obtention de mutants de Phaffia rhodozyma producteur de β-carotène selon l'invention, caractérisé en ce que :
1) on traite une souche sauvage de Phaffia rhodozyma en culture par un agent mutagène physique ou chimique, et
2)on sélectionne les clones de couleur jaune obtenus après le traitement de l'étape 1).

Comme on l'a mentionné dans un mode de réalisation, à l'étape 1), on traite la souche sauvage de Phaffia rhodozyma par un agent mutagène consistant dans des rayons ultra-violets.

La présente invention a aussi pour objet un procédé de production de β-carotène par fermentation d'une souche mutante de levure de Phaffia rhodozyma selon l'invention.

On obtient une augmentation du rendement de production en β-carotène quand le milieu de culture est enrichi en acide mévalonique. (l'acide mévalonique (AMV) est en effet un précurseur du β-carotène dans la voie de biosynthèse de l'astaxanthine).

La présente invention a aussi pour objet l'utilisation de biomasse, éventuellement déshydratée, d'un mutant de levure Phaffia rhodozyma selon l'invention, comme supplément de β-carotène dans la préparation de produits alimentaires ou diététiques, cosmétiques, parapharmaceutiques ou pharmaceutiques.

La présente invention fournit en outre un procédé de production de β-ionone par chauffage de cellules d'un mutant de levure Phaffia rhodozyma selon l'invention.

La présente invention a enfin pour objet l'utilisation de la biomasse d'une levure Phaffia rhodozyma selon l'invention comme source de β-ionone notamment dans la préparation de produits alimentaires ou diététiques, cosmétiques, parapharmaceutiques ou pharmaceutiques.

D'autres caractéristiques et avantages de la présente invention apparaîtront à la lumière des exemples qui vont suivre.

La Figure 1 représente la voie de biosynthèse de l'astaxanthine chez Phaffia rhodozyma.

La Figure 2 représente le spectre d'absorption de l'extrait de la souche PG 104 (A) et du produit de référence (β-carotène pur, Sygma) (B).

La Figure 3 représente l'analyse HPLC des extraits de la souche sauvage CBS 6938 de Phaffia rhodozyma (A), du mutant PG 104 (B) et des produits de référence alpha-carotène, (Sygma) et β-carotène.

### EXEMPLE 1

### Obtention des mutants

Les cellules de la souche sauvage CBS 6938 de Phaffia rhodozyma sont cultivées sur un milieu liquide (YPG) contenant 1% d'extrait de levure, 1% de bactopeptone et 2% de glucose, sous agitation (150 rpm) à 25°C. Les cultures âgées de 72 heures sont ceutrifugées 5 min à 3.000 g et les cellules lavées deux fois à l'eau distillée et traitées aux UV pendant 50 sec à 35 cm d'une lampe germicide, ce qui correspond à une dose de 1.789 J x s⁻¹ x cm². Le taux de survie est d'environ 10%. Les cellules traitées sont étalées sur des boîtes de YPG solide et incubées à 25°C pendant 72 heures. Sur 5 x 10⁴ colonies examinées, quelques colonies blanches ont été repérées, ainsi que deux colonies de couleur jaune (PG 104 et PG 126) contrastant avec la couleur rouge des colonies sauvages. Le mutant PG 104 a fait l'objet des expérimentations ultérieures.

### EXEMPLE 2

### Identification des caroténoïdes du mutant PG 104

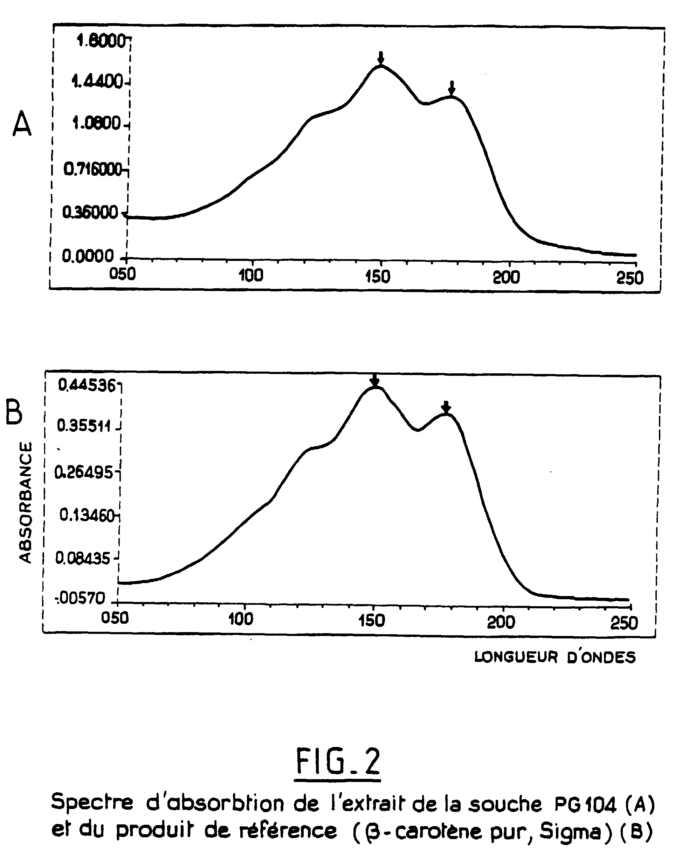

100 ml de milieu SD (50,67% de bacto-yeast nitrogen base sans acides aminés et 2% de glucose) sont placés dans des fioles Erlenmayer de 1.000 ml et inoculés avec 10⁶ cellules/ml de la souche PG 104 prélevées d'une préculture en YPG. Les cultures sont incubées à 25°C sous agitation (150 rpm) et au bout de 72 heures, les cellules sont récoltées par centrifugation et soumises à un traitement lytique : à 200 ml de suspension cellulaire dans du tampon citrate-phosphate 0,1 M, pH 5,6, contenant 10⁷ cellules/ml, on ajoute 6 mg/ml de préparation enzymatique (Novozym 234) ; après une incubation de 60 min à 25°C sous agitation faible, l'examen microscopique montre une lyse des parois cellulaires à pratiquement 100%. La préparation de protoplastes est alors traitée à l'acétone et les caroténoïdes sont extraits à l'éther de pétrole. Le spectre UV-VIS de l'extrait (Fig. 2) est identique à celui du produit de référence (β-carotène pur, Sygma).

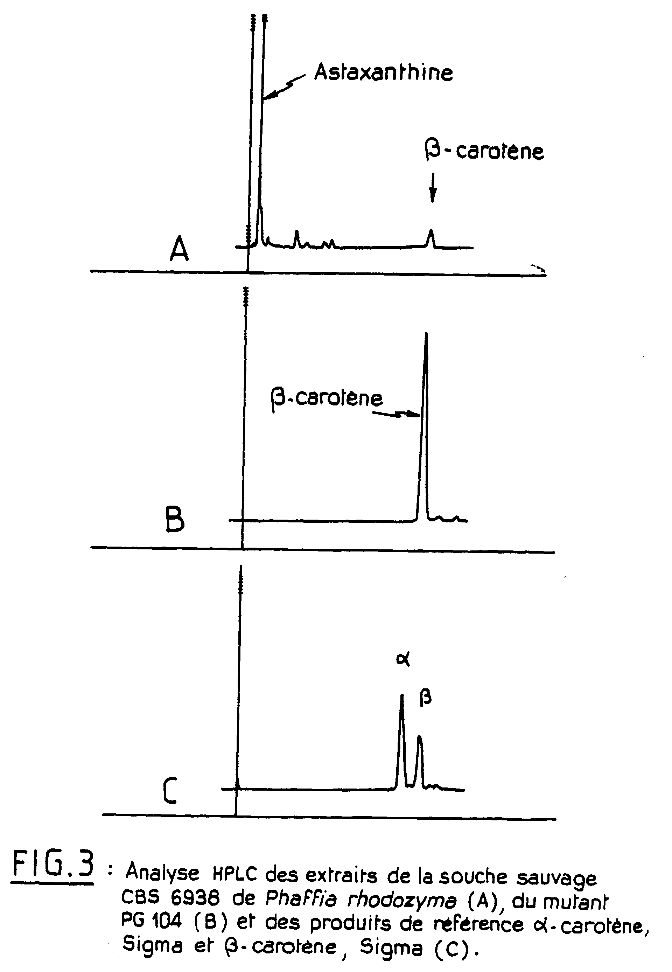

L'analyse par HPLC (Fig. 3) sur colonne Microbondapak RP 18 (Waters), avec comme phase mobile du méthanol, montre dans le cas du mutant PG 104 la présence d'un seul pic, correspondant à celui du β-carotène pur, contrairement à la souche sauvage qui contient de l'astaxanthine comme pic majeur, mais aussi, en faibles quantités, d'autres intermédiaires probables de la voie des caroténoïdes.

Une comparaison avec les proportions relatives de différents caroténoïdes chez une souche sauvage (Tableau I) montre clairement que le mutant PG 104 produit pratiquement en exclusivité du β-carotène. Il correspond probablement à un blocage mutationnel au niveau de l'étape de conversion du β-carotène en échinénone.

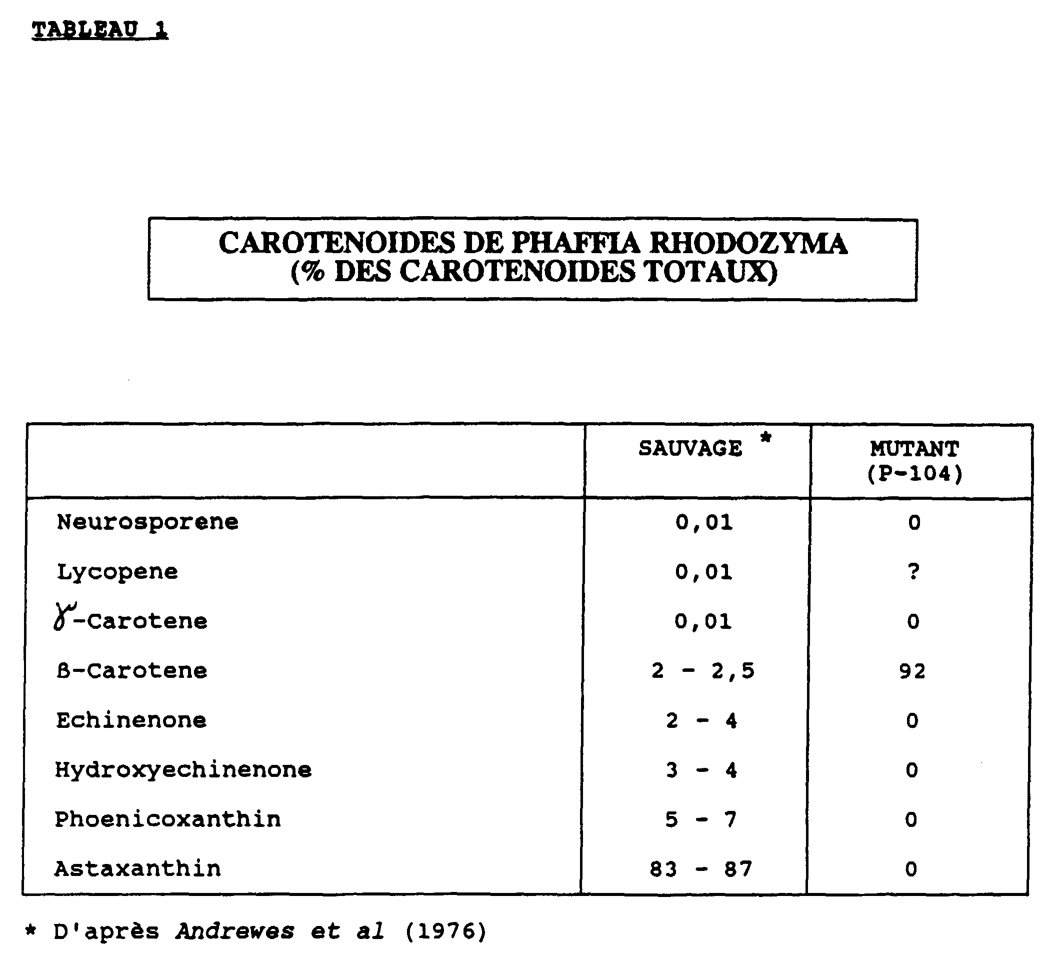

### EXEMPLE 3

### Production de β-carotène avec le mutant PG 104

Différents milieux et conditions de culture, ainsi que l'effet de certains suppléments sur la biosynthèse de β-carotène par PG 104 ont été testés, afin d'établir les paramètres permettant d'augmenter cette production. Les résultats sont présentés dans le tableau II.

A la suite de ces tests, les paramètres culturaux suivants ont été choisis pour une production optimum de β-carotène :
- Milieu de culture : SGly-HC-AMV
- Inoculum : 10⁶ cpm
- Volume : 1/10 du volume de la fiole
- Température : 25°C
- Agitation : 150 rpm

Dans ces conditions, on obtient au bout de 96 heures jusqu'à 10 mg de β-carotène par litre de culture.

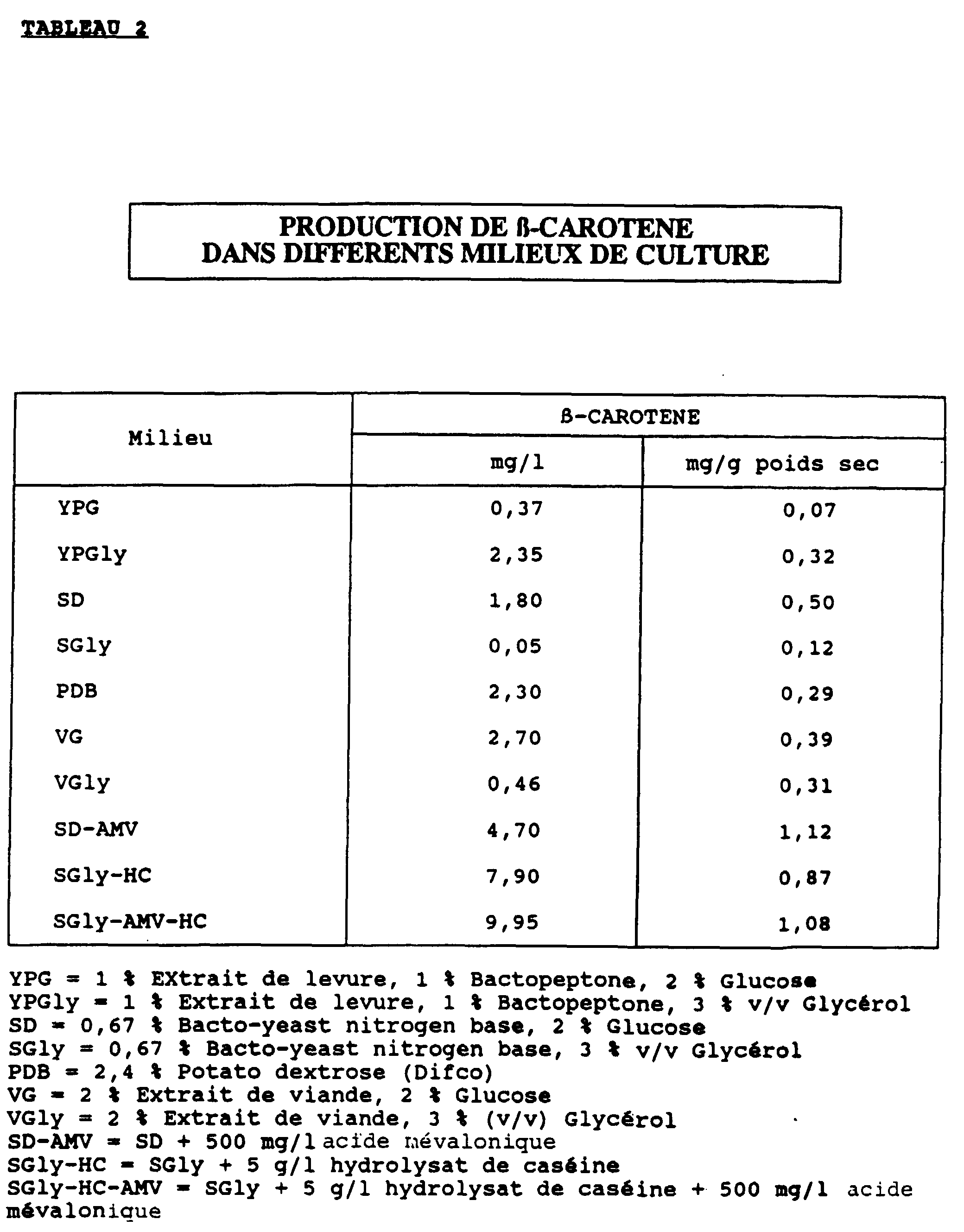

### EXEMPLE 4

### Préparation brute de β-carotène à partir de cellules du mutant PG 104 de Phaffia rhodozyma

Les cellules de PG 104 cultivées dans les conditions standard sont récoltées par centrifugation et lavées une fois à l'eau distillée. A 1 g (poids humide) de cellules, on ajoute 100 ml d'éthanol absolu et la suspension est agitée 1 heure à 150 rpm. Les cellules sont récoltées par centrifugation et le culot est réparti en couches de 1-2 mm d'épaisseur sur des boîtes de *Petri* et séché 24 heures à température ambiante. On obtient une poudre qui contient 2 mg de β-carotène par gramme. Des aliquots de cette poudre sont étalés sur des boîtes de YPG solide et l'absence de croissance au bout de 2 semaines montre que la préparation ne contient aucune cellule vivante.

Cettre préparation peut être utilisée comme supplément de β-carotène dans des jus de fruits, et tout autre produit alimentaire, ainsi que dans des produits cosmétiques et dans l'alimentation animale.

### EXEMPLE 5

### Préparation riche en β-carotène à partir de cellules de Phaffia rhodozyma PG 104

Les cellules récoltées dans 100 ml de culture sont traitées au Novozym (comme décrit dans l'exemple 2) et extraites 5 fois à l'acétone. Les extraits sont réunis et les débris cellulaires sont éliminés par centrifugation. Le surnageant acétonique est placé dans une ampoule à décanter ; 10 ml d'éther de pétrole et 1 ml de NaCl saturé sont ajoutés. On récupère la phase supérieure (éther de pétrole) fortement coloré ; la phase acétonique est extraite une deuxième fois à l'éther de pétrole et les extraits en éther sont réunis, filtrés, séchés sur Na₂ SO₄ anhydre et repris dans 1 ml d'éther de pétrole. On confirme l'identité du caroténoïde extrait par spectrophotométrie. L'extrait final contient 1 g de β-carotène par litre.

### EXEMPLE 6

### Production de β-ionone au cours de l'élaboration de boissons distillées

La β-ionone est un arôme cher utilisé dans l'industrie alimentaire. Du moût de malt de distillerie (densité initiale 1060), est fermenté avec une souche de distillerie traditionnelle, jusqu'à une densité de 997. Avant la distillation, on ajoute 1 g de cellules de Phaffia rhodozyma PG 104 traitées préalablement au Novozym. Dans le low wine obtenu après distillation, on met en évidence par GC-MS la présence de traces de β-ionon e, absentes dans le produit témoin.

### BIBLIOGRAPHIE

Ben-Amotz A., Avron M. (1983) On factors which determine massive β-carotene accumulation in the halotolerant alga *Dunaliella bardawil.* Plant Physiol. 72 : 593-597.

Murillo-Araujo F.J., Calderon I.L., Lopez-Diaz I., Cerda-Olmedo E. (1978) Carotene superproducing strains of Phycomyces. Appl. Environ. Microbiol.36 : 639-642.

Ninet L., Renaut J. (1979) In "Microbial Technology", H.J. Peppler & D. Perlman eds., 2nd Ed. Vol. 1, pp. 529-544, Academic Press, New York.

Igene Biotechnology, Inc.(1989) Processes for in vivo production of astaxanthin and *Phaffia rhodozyma* yeast of enhanced astaxanthin content. Brevet WO 91/02060.

Andrewes A.G., Phaff H.J., Starr M.P.(1976) Carotenoids of *Phaffia rhodozyma,* a red-pigmented fermenting yeast. Phytochemistry 15: 1003-1007.

## Revendications

1. Mutants de Phaffia rhodozyma bloqués dans l'étape de conversion du β-carotène dans la voie de bio-synthèse de l'astaxanthine.

2. Procédé d'obtention de mutants de Phaffia rhodozyma producteurs de β-carotène selon la revendication 1, caractérisé en ce que :
1) on traite une souche sauvage de Phaffia rhodozyma en culture par un agent mutagène physique ou chimique, et
2) on sélectionne les clones de couleur jaune obtenus après le traitement de l'étape 1).

3. Procédé selon la revendication 2 caractérisé en ce que, à l'étape 1), on traite la souche sauvage de Phaffia rhodozyma par un agent mutagène consistant dans des ultra-violets.

4. Procédé de production de β-carotène par fermentation d'une souche mutante de levure de Phaffia rhodozyma selon la revendication 1.

5. Procédé selon la revendication 4, caractérisé en ce que le milieu de culture est enrichi en acide mévalonique.

6. Utilisation de biomasse, éventuellement déshydratée, d'un mutant de levure Phaffia rhodozyma selon la revendication 1, comme supplément de β-carotène dans la préparation de produits alimentaires ou diététiques, cosmétiques, parapharmaceutiques ou pharmaceutiques.

7. Procédé de production de β-ionone par chauffage de cellules d'un mutant de levure Phaffia rhodozyma selon la revendication 1.

8. Utilisation de la biomasse d'une levure Phaffia rhodozyma selon la revendication 1 comme source de β-ionone notamment dans la préparation de produits alimentaires ou diététiques, cosmétiques, parapharmaceutiques ou pharmaceutiques.

## Claims

1. Phaffia rhodozyma mutants blocked in the step of conversion of β-carotene in the pathway of biosynthesis of astanxanthin.

2. Process for obtaining Phaffia rhodozyma mutants producing β-carotene according to Claim 1, characterized in that:
1) a wild-type strain of Phaffia rhodozyma in culture is treated with a physical or chemical mutagenic agent, and
2) the yellow-coloured clones obtained after the treatment of step 1) are selected.

3. Process according to Claim 2, characterized in that, in step 1), the wild-type strain of Phaffia rhodozyma is treated with a mutagenic agent consisting of ultraviolet rays.

4. Process for producing β-carotene, by fermentation of a mutant strain of Phaffia rhodozyma yeast according to Claim 1.

5. Process according to Claim 4, characterized in that the culture medium is enriched in mevalonic acid.

6. Use of biomass, where appropriate dehydrated, of a mutant of Phaffia rhodozyma yeast according to Claim 1, as a β-carotene supplement in the preparation of foodstuffs or dietary, cosmetic, parapharmaceutical or pharmaceutical products.

7. Process for producing β-ionone, by heating cells of a mutant of Phaffia rhodozyma yeast according to Claim 1.

8. Use of the biomass of a Phaffia rhodozyma yeast according to Claim 1 as a source of β-ionone, in particular in the preparation of foodstuffs or dietary, cosmetic, parapharmaceutical or pharmaceutical products.

## Patentansprüche

1. Mutanten von Phaffia rhodozyma, die in dem Schritt der Umwandlung des β-Karotins in dem Weg der Biosynthese von Astaxanthin blockiert sind.

2. Verfahren zur Gewinnung von β-Karotin produzierenden Mutanten von Phaffia rhodozyma nach Anspruch 1, dadurch gekennzeichnet, daß:
1) man einen Wildstamm von Phaffia rhodozyma in Kultur durch ein physikalisches oder chemisches mutagenes Mittel behandelt und
2) man die Klone gelber Farbe, die nach der Behandlung des Schritts 1) erhalten werden, selektiert.

3. Verfahren nach Anspruch 2, dadurch gekennzeichnet, daß man in Schritt 1) den Wildatamm von Phaffia rhodozyma durch ein mutagenes Mittel, das in ultravioletten Strahlen besteht, behandelt.

4. Verfahren zur Herstellung von β-Karotin durch Fermentation eines mutierten Hefestamms von Phaffia rhodozyma nach Anspruch 1.

5. Verfahren nach Anspruch 4, dadurch gekennzeichnet, daß das Kulturmedium an Mevalonsäure angereichert wird.

6. Verwendung von gegebenenfalls dehydratisierter Biomasse einer Mutante der Hefe Phaffia rhodozyma nach Anspruch 1 als β-Karotin-Zusatz bei der Herstellung von Lebensmitteln oder dietätischen, kosmetischen, parapharmazeutischen oder pharmazeutischen Produkten.

7. Verfahren zur Herstellung von β-Ionon durch Erwärmen von Zellen einer Mutante der Hefe Phaffia rhodozyma nach Anspruch 1.

8. Verwendung der Biomasse von einer Hefe Phaffia rhodozyma nach Anspruch 1 als Quelle von β-Ionon insbesondere bei der Herstellung von Lebensmitteln oder dietätischen, kosmetischen, parapharmazeutischen oder pharmazeutischen Produkten.
